**Europäisches Patentamt**

**European Patent Office**

**Office européen des brevets**

(11) Veröffentlichungsnummer: **0 270 794 B1**

# (12) EUROPÄISCHE PATENTSCHRIFT

(45) Veröffentlichungstag der Patentschrift: **03.06.92**

(21) Anmeldenummer: **87115472.0**

(22) Anmeldetag: **22.10.87**

(51) Int. Cl.5: **A61M 1/14**

---

(54) **Hämodialysevorrichtung mit Sterilisiereinrichtung.**

---

(30) Priorität: **08.12.86 DE 3641843**

(43) Veröffentlichungstag der Anmeldung:
**15.06.88 Patentblatt 88/24**

(45) Bekanntmachung des Hinweises auf die
Patenterteilung:
**03.06.92 Patentblatt 92/23**

(84) Benannte Vertragsstaaten:
**BE CH DE ES FR GB IT LI NL SE**

(56) Entgegenhaltungen:
**DE-A- 3 407 147**
**DE-A- 3 444 671**

(73) Patentinhaber: **Fresenius AG**
**Gluckensteinweg 5**
**W-6380 Bad Homburg v.d.H.(DE)**

(72) Erfinder: **Polaschegg, Hans-Dietrich, Dr.**
**Grünwiesenweg 9**
**W-6370 Oberursel(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**W-6200 Wiesbaden(DE)**

## Beschreibung

Die Erfindung betrifft eine Hämodialysevorrichtung mit Sterilisiereinrichtung für die Dialysierflüssigkeit, mit einem Dialysator, der durch eine Membran in zwei Kammern geteilt ist, wobei die erste Kammer in einen Dialysierflüssigkeitsweg und die zweite Kammer in einen Blutweg geschaltet ist, der Dialysierflüssigkeitsweg eine Zuleitung, die sich von einer Einrichtung zur Bereitstellung von Dialysierflüssigkeit bis zum Dialysator erstreckt und in die ein erstes Absperrorgan eingeschaltet ist, und eine Ableitung aufweist, die sich vom Dialysator zum Abfluß erstreckt und in die ein zweites Absperrorgan eingeschaltet ist, mit einer Pumpe zum Fördern der Dialysierflüssigkeit im Dialysierflüssigkeitsweg, einer zwischen den Absperrorganen im Dialysierflüssigkeitsweg vorgesehenen Ultrafiltrationseinichtung, mit einer die Zuleitung mit der Ableitung des Dialysierflüssigkeitswegs verbindenden Bypassleitung, in die ein Bypassventil eingeschaltet ist, und mit einem Sterilfilter, das durch eine Keime zurückhaltende Membran in eine erste Kammer und eine zweite Kammer geteilt ist, mit einem ersten Abschnitt der Zuleitung der Dialysierflüssigkeit zur ersten Kammer des Sterilfilters und einem zweiten Abschnitt der Zuleitung der Dialysierflüssigkeit von der zweiten Kammer des Sterilfilters zum Dialysator.

Bei der Hämodialyse wird Blut an der Membran eines Dialysators entlang geführt, wobei auf der anderen Seite der Membran eine Dialysierflüssigkeit vorbeigeführt wird, die durch die Poren der Membran hindurch die aus dem Blut zu entfernenden Stoffwechselprodukte aufnimmt. Zusätzlich kann durch Anlegen von Unterdruck an die Membran Wasser mittels Ultrafiltration aus dem Blut entfernt werden.

Die Dialysierflüssigkeit wird üblicherweise on line aus Frischwasser und einem Elektrolytkonzentrat hergestellt, wobei letzteres eigensteril ist und Frischwasser üblicherweise keine Keime aufweist. Es ist jedoch nicht sichergestellt, daß die so hergestellte Dialysierflüssigkeit absolut steril ist.

Insofern wurde vorgeschlagen, stromauf des Dialysators in den Dialysierflüssigkeitsweg ein Sterilfilter einzuschalten, um dem Dialysator eine absolut sterile Dialysierflüssigkeit zuzuführen.

In ähnlicher Weise wurde aus der nicht absolut sterilen Dialysierflüssigkeit eine völlig sterile Substitutionslösung für die Hämofiltration hergestellt, wobei die von der Dialysierflüssigkeitsleitung abgehende Substituatleitung wenigstens ein Sterilfilter aufweist. Derartige Systeme sind beispielsweise in der DE-OS 34 44 671, der EP-OS 42 939 und Trans.Am.Soc.Artif.Intern.Organs.-(ASAIO), Bd.24 (1978), S.465-467 und Bd.25 (1979), S.404-408 beschrieben.

Bisher wird jedoch nur ausnahmsweise ein derartiger Sterilfilter in Hämodialysegeräten eingeschaltet, da die derzeit am Markt befindlichen Dialysatoren in ihrer Filtrationseigenschaft derart beschränkt sind, daß sie nur die Filtration von der Blutseite auf die Dialysierflüssigkeitsseite und nicht umgekehrt zulassen.

Die Entwicklung von leistungsfähigen Dialysatoren einerseits und von Dialysegeräten mit Ultrafiltrationskontrolle andererseits hat dazu geführt, daß es während der Hämodialyse zu einem Druckgefälle von der Dialysierflüssigkeit zum Blut kommen kann, was zur Rückfiltration führen kann.

Hochleistungsfähige Dialysatoren, wie z.B. der F60-Dialysator der Anmelderin, besitzen einen hohen Ultrafiltrationskoeffizienten, d.h. es bedarf nur eines geringen Transmembrandruckes, um den bei der Behandlung der chronischen und akuten Niereninsuffizienz üblicherweise notwendigen Flüssigkeitsentzug zu erzielen.

Moderne Hämodialysegeräte, wie das Gerät A2008C der Anmelderin, besitzen Einrichtungen zur Kontrolle des Flüssigkeitsentzuges, wobei es besonders vorteilhaft ist, daß dieses Gerät eine Behandlung ohne Flüssigkeitsentzug erlaubt.

Aufgrund der Strömungswiderstände treten dabei Zonen im Dialysator auf, bei denen der Druck in der Dialysierflüssigkeit größer als der Druck auf der Blutseite ist. Es kommt dadurch zu der oben genannten Rückfiltration.

Obwohl dieses Phänomen seit Jahren bekannt ist und mittlerweile hunderttausende Behandlungen komplikationslos durchgeführt wurden, besteht die Befürchtung, daß es bei einer Ruptur innerhalb einer Membranzone des Dialysators und bei Rückfiltration zu einer Kontamination des Blutes kommt, da die Dialysierflüssigkeit in der Regel nicht völlig steril ist.

Eine Maßnahme, Sicherheit gegen eine solche - wie vorstehend erwähnt - nicht auszuschließende Möglichkeit zu schaffen, besteht darin, die Dialysierflüssigkeit unmittelbar vor dem Dialysator durch einen Filter zu leiten, der Keime und Pyrogene (fiebererregende Stoffe) zurückhält. Zwar kann auch ein solcher Filter während der Behandlung defekt werden. Dies ist aber solange irrelevant, als der Dialysator in Takt bleibt. Die Wahrscheinlichkeit, daß während einer Behandlung sowohl der Dialysator als auch das Sterilfilter einen Defekt aufweisen, ist vernachlässigbar gering. Das damit verbundene Restrisiko entspricht dem in der Hämodialyse üblichen Risiko. Wesentlich ist allerdings die Einschränkung auf den Zeitraum einer einzigen Hämodialyse-Behandlung. Dies macht es notwendig, daß der Sterilfilter rgelmäßig vor jeder Behandlung getestet wird. Erfolgt dies nicht, so kann mit einem nicht intakten Filter über einen längeren Zeitraum gearbeitet werden, ohne daß dies bemerkt wird. Ein Defekt am Hämodialysator führt dann automatisch zur Gefährdung. Des weiteren soll das Sterilfilter möglichst optimal betrieben werden, was eine regelmäßige Reinigung voraussetzt.

Eine Anordnung der genannten Art weist allerdings folgende Nachteile für den Routinebetrieb auf, bei der der Filter so lange als möglich benutzt werden soll:

Die sogenannte "dead end"-Anordnung des Filters führt dazu, daß sich im Laufe der Zeit Partikel und andere Substanzen, z.B. Pyrogene vor dem Filter ansammeln und diesen verstopfen können. Dies ist insbesondere dann gefährlich, wenn durch eine Ruptur diese Substanzen schlagartig in den Sterilbereich eingeschleppt werden. Bei dieser "dead end"-Anordnung wird der Filter durch stromauf erzeugten Druck in die Filterkammer gefördert, wobei die eingeschleppten Keime und Pyrogene die Poren der Membran zusetzen. Die gezeigte Anordnung erlaubt es nicht, das Sterilfilter vor jeder Behandlung mit einfachen Mitteln auf Intaktheit zu prüfen.

Der Erfindung liegt daher die Aufgabe zugrunde, die Dialysevorrichtung der eingangs erwähnten Art so fortzubilden, daß ein Zusetzen des Sterilfilters mit Keimen oder Pyrogenen während der Dialysebehandlung im wesentlichen verhindert wird.

Die Lösung der Aufgabe erfolgt dadurch, daß der Auslaß der ersten Kammer des Sterilfilters mit der zur Ableitung führenden Bypassleitung verbunden ist.

Erfindungsgemäß wird die Anordnung eines Sterilfilters vorgeschlagen, dessen Ausgang, der üblicherweise verschlossen ist, mit der zum Abfluß führenden Leitung verbunden ist. Dabei kann das Bypassventil in vorbestimmten Abständen sowohl während der Behandlung als auch während des Spülbetriebs der gesamten Anordnung geöffnet werden. Wenn bei der Behandlung das Bypassventil geöffnet wird, fließt die Dialysierflüssigkeit aus der Primärkammer des Sterilfilters ab und reißt dort die an der Membran befindlichen Pyrogene und Teilchen in den Abfluß mit. Es erfolgt dabei keine sterilisierend wirkende Filtration der Dialysierflüssigkeit durch die Poren der Membran, da der im nachgeschalteten Dialysator vorliegende Flußwiderstand in Verbindung mit dem Verstopfungsgrad der Poren eine solche Strömung verhindert.

Nach einer bestimmten Spüldauer wird dann das Bypassventil wieder geschlossen, so daß erneut die Dialysebehandlung erfolgen kann.

Des weiteren kann das Sterilfilter automatisch nach jeder Behandlung mit Wasser und Desinfektionslösung gespült und danach auf eine Ruptur getestet werden.

Zu diesem Zweck ist das Sterilfilter gemäß dieser bevorzugten Ausführungsform wieder in den Dialysierflüssigkeitskreislauf geschaltet (die Primärseite im sogen.

Bypasskreis und die Sekundärseite im sogen. Dialysierflüssigkeitskreis). Da bei jedem Desinfektions- und Spülvorgang des Hämodialysegeräts automatisch zwischen Bypass- und Dialysemodus umgeschaltet wird, erfolgt regelmäßig die Spülung der Primärseite des Sterilfilters. Des weiteren kann die Membran des Sterilfilters einfach auf ihre Intaktheit geprüft werden. Dabei wird die Dialysevorrichtung bei geschlossenem Bypassventil in den Dialysebetrieb geschaltet und die Bypassleitung zwischen dem Sterilfilter und dem Bypassventil belüftet. Zu diesem Zweck ist eine Belüftungseinrichtung vorgesehen, die beispielsweise als von der Bypassleitung abgehende Leitung ausgebildet sein kann, deren Ende mit einem Belüftungsventil verschlossen ist. Dieses Ventil wird zur Belüftung geöffnet, so daß nur noch Luft in den Kreislauf eingeführt wird, da die Dialysierflüssigkeitszuführung unterbrochen ist. An dem so vorbereiteten Gerät kann dann ein Druckhaltetest durchgeführt werden, mit dem die Intaktheit des Flüssigkeitsbilanziersystems des Geräts und des Sterilfilters überprüft werden kann. Bei diesem Druckhaltetest wird dem geschlossenen Kreislauf mit Hilfe der Ultrafiltratpumpe Flüssigkeit entzogen, wobei diese Flüssigkeit zunächst aus dem Bypassteil durch die zugleich zugeführte Luft verdrängt wird. Die Verdrängung der Dialysierflüssigkeit ist dann beendet, wenn die gesamte Primärseite des Sterilfilters mit Luft gefüllt ist. Die Membran des Sterilfilters bildet infolge ihrer Benetzung mit Wasser eine für Luft undurchdringliche Barriere mit der Folge, daß sich bei weiterer Abförderung von Dialysierflüssigkeit auf der Sekundärseite des Sterilfilters schnell ein Unterdruck aufbaut, der auch nach dem Abschalten der Ultrafiltratpumpe über längere Zeit stabil bleibt. Der Druckwert, bei dem die Ultrafiltratpumpe abgeschaltet wird, kann an einem Steuergerät eingestellt werden, das nach Erreichen dieses Druckwerts die Ultrafiltratpumpe abschaltet und anschließend an einem Druckmanometer den zeitlichen Verlauf der Druckwerte verfolgt.

Dieser Druckwert wird im wesentlichen über eine bestimmte Zeitdauer stabil bleiben, wenn die Membran intakt ist, also keine Luft die Membran durchdringen kann. Weist die Membran hingegen ein Leck auf, so wird Luft nachströmen, was zu einem negativen Verlauf des Druckhaltetests führt.

Infolgedessen gibt dieser Druckhaltetest zunächst einen Hinweis auf einen Defekt im gesamten Bilanziersystem oder am Sterilfilter. Man kann in einem nachfolgenden zweiten Testschritt dann zwischen diesen beiden Möglichkeiten differenzieren, indem die Belüftung des Sterilfilters aufgehoben, d.h. das Belüftungsventil geschlossen wird und der Test wiederholt wird. In einem solchen Schritt wird dann der geschlossene Kreislauf, also das Bilanziersystem, getestet, wie dies bei der A2008C der Anmelderin bereits üblich ist.

Um das Sterilfilter zwangsläufig nach diesem Testvorgang zu entlüften, wird im Primärkreis die Flüssigkeitszuleitung vorteilhafterweise unten und die Ableitung vorteilhafterweise oben liegend angeordnet. Im Sekundärkreislauf werden entweder beide Ausgänge miteinander verbunden oder aber der untere wird verschlossen und nur der obere Ausgang wird benutzt.

Gemäß einer ersten Ausführungsform kann die zum Test notwendige Belüftung der Bypassleitung manuell erfolgen. Vorteilhafterweise erfolgt die Belüftung durch eine Einrichtung, die eine sterile Belüftung dieses Vorrichtungsteils erlaubt, um nicht schwer zu desinfizierende Totzonen entstehen zu lassen.

Zu diesem Zweck wird innerhalb eines druckstabilen Gehäuses eine hydrophobe, mikroporöse und luftdurchlässige Membran in dem Gehäuse vorgesehen, die mit der Bypassleitung in Strömungsverbindung steht. Von dem Gehäuse selbst geht ein Entlüftungsstutzen ab, dessen Ende mit dem Belüftungsventil verschlossen ist.

Sofern sichergestellt ist, daß bei der Hämodialyse dieser Bereich stets einen der Umgebung positiven Druck aufweist, so wird dieser Bereich stets luftfrei sein, d.h. das Belüftungsventil muß nicht notwendigerweise vorgesehen sein. Beim Druckhaltetest hingegen wird ein Unterdruck erzeugt, der den Eintritt von Luft durch die Poren des Schlauches erlaubt.

Ist jedoch nicht gewährleistet, daß stets ein Überdruck im Bereich dieser Belüftungseinrichtung vorliegt, so ist an das Gehäuse das vorstehend erwähnte Belüftungsventil angeschlossen. Während des Tests wird dann dieses Ventil geöffnet, um die Luft zur Bypassleitung zuströmen zu lassen.

Gemäß einer weiteren vorteilhaften Ausführungsform wird die Anordnung vorteilhafterweise so gewählt, daß sich eventuell bildendes Kondenswasser im Außenraum (Luftraum) beim Öffnen des Belüftungsventils abfließen kann.

Weitere Vorteile der Erfindung werden anhand eines Ausführungsbeispiels erläutert.

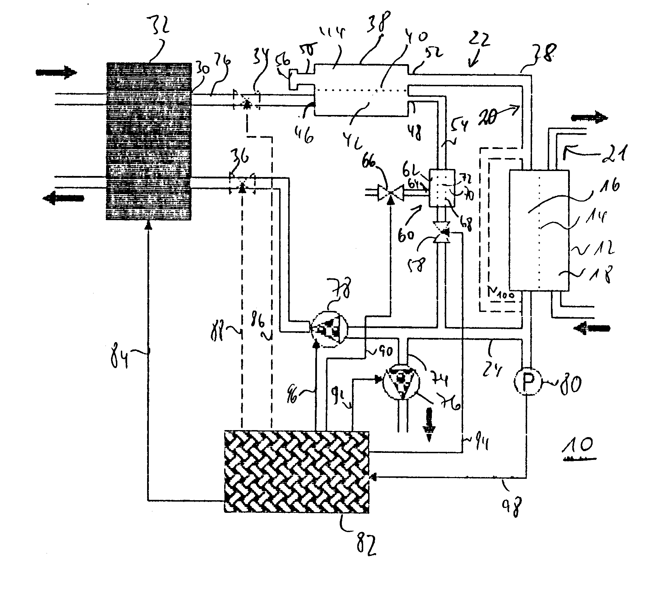

Es zeigt
die Figur schematisch eine Hämodialysevorrichtung mit Sterilisierungseinrichtung für die Dialysierflüssigkeit.

In der Figur ist mit 10 ein Hämodialysegerät gezeigt, das einen üblichen Dialysator 12 aufweist, der durch eine Membran 14 in eine von Dialysierflüssigkeit durchflossene Kammer 16 und in eine mit Blut durchflossene Kammer 18 getrennt ist.

Die Kammer 16 ist in einen Dialysierflüssigkeitsweg 20 eingeschaltet, der aus einer Zuleitung 22 und einer Ableitung 24 besteht. Andererseits ist die Kammer 18 in einen Blutweg 21 geschaltet.

Wie aus der Figur ersichtlich ist, besteht die Ableitung 22 aus einem ersten Zuleitungsabschnitt 26 und einem zweiten Zuleitungsabschnitt 28. Der erste Leitungsabschnitt 26 ist an seinem einen Ende 30 mit einer Bilanzkammer 32 verbunden, wie sie beispielsweise bei der A2008C der Anmelderin verwirklicht ist und in der DE-OS 28 38 414 beschrieben ist, worauf aus Offenbarungsgründen ausdrücklich Bezug genommen wird.

Diese Bilanzkammer ist durch eine nichtgezeigte flexible Wand in zwei Hälften getrennt, wobei die eine Hälfte mit der Zuleitung 22 verbunden ist und die andere Hälfte mit der Ableitung 24 verbunden ist. Des weiteren ist aus Übersichtlichkeitsgründen eine zweite Bilanzkammer weggelassen worden, die mit frischer Dialysierflüssigkeit bzw. Spül- oder Desinfektionslösung gefüllt wird. Dieser Füll- bzw. Entleerungsvorgang ist durch die beiden Teile, die in der Zeichnung links neben der Bilanzkammer dargestellt sind, versinnbildlicht. Demzufolge wird also frische Dialysierflüssigkeit aus einer nichtgezeigten Dialysierflüssigkeitsquelle der Bilanzkammer 32 zugeführt und am Auslaß wieder nach dem Durchlauf durch den Dialysator 12 entfernt. Anstelle der Bilanzkammer 32 kann gemäß einer weiteren Ausführungsform, sofern eine übliche Single-Pass-Vorrichtung verwendet wird, in der Zuleitung 22, insbesondere dem ersten Zuleitungsabschnitt 26, ein erstes Absperrventil 34 angeordnet sein, wie dies in der Figur strichliert dargestellt ist.

Des weiteren kann in der Ableitung 24 ein zweites Absperrventil 36 angeordnet sein, mit der Folge, daß zwischen den beiden Ventilen 34 und 36 bzw. zwischen den Bilanzkammerhälften der Bilanzkammer 32 ein abgeschlossenes System aufgebaut werden kann. Sowohl die Bilanzkammer 32 als auch die beiden Absperrventile 34 und 36 stellen dabei Absperrorgane im Sinne der Erfindung dar.

In die Zuleitung 22 ist ein Sterilfilter 38 eingeschaltet, das eine Membran 40 aufweist, das das Sterilfilter 38 in eine erste Kammer 42 und eine zweite Kammer 44 trennt. Die beiden Kammern 42 und 44 haben jeweils Anschlußeinrichtungen 46 bis 52, da vorteilhafterweise ein handelsüblicher Sterilfilter eingesetzt wird, der üblicherweise über jeweils zwei Zugänge und zwei Abgänge verfügt. So kann vorteilhafterweise auch ein Dialysefilter als Sterilfilter eingesetzt werden, beispielsweise der F60-Dialysefilter der Anmelderin.

Der Einlaß 46 ist dabei mit dem anderen Ende des ersten Zuleitungsabschnitts 26 verbunden. Der Auslaß 48 der ersten Kammer 42 des Sterilfilters 38 ist mit der Bypassleitung 54 verbunden, während der Auslaß 52 der zweiten Kammer 44 des Sterilfilters 38 mit dem einen Ende des zweiten Zuleitungsabschnitts 28 verbunden ist. Das andere Ende des Zuleitungsabschnitts 28 ist im Dialysebetrieb mit dem Eingang der ersten Kammer 16 des Dialysators 12 verbunden. Schließlich ist der Zugang 50 der zweiten Kammer 44 des Sterilfilters 38 mit einem Stopfen 56 verschlossen.

Die Membran 40 des Sterilfilters weist eine solche Porengröße auf, daß sie die durch sie geförderte Dialysierflüssigkeit sterilisiert, d.h. Keime oder Pyrogene an der Membranoberfläche zurückhält. Insofern weist diese Membran eine Porengröße von höchstens 0,2 μm auf.

Die Bypassleitung 54 ist an ihrem anderen Ende mit der Ableitung 24 verbunden.

Des weiteren ist in die Bypassleitung 54 ein Bypassventil 58 eingeschaltet, vor dem sich stromauf, d.h. in Richtung auf das Sterilfilter 38, eine Belüftungseinrichtung 60 befindet.

Gemäß einer ersten Ausführungsform weist diese Belüftungseinrichtung 60 ein Gehäuse 62 auf, das an seiner Umfangsfläche einen Stutzen 64 aufweist, dessen Ende mit einem Belüftungsventil 66 verschlossen ist. Des weiteren weist das Gehäuse 62 im Inneren eine vorteilhafterweise schlauchförmig ausgebildete Membran 68 auf, die das Gehäuse in eine erste Kammer 70, die mit dem Stutzen 64 in Strömungsverbindung steht, und in eine zweite Kammer 72 teilt, die mit der Bypassleitung 54 in Strömungsverbindung steht. Die schlauchförmige Membran 68 ist vorteilhafterweise hydrophob augebildet, so daß durch die Poren der Membran kein Wasser dringen kann. Des weiteren ist die Membran 68 derart mikroporös, daß keine Keime in die Bypassleitung eindringen können. Insofern weist diese Membran 68 vorteilhafterweise eine Porengröße von 0,2 μm oder darunter auf.

Von der Ableitung 24 geht weiterhin eine Ultrafiltratleitung 74 ab, in die eine Ultrafiltrationspumpe 76 eingeschaltet ist.

Des weiteren ist in die Abzugsleitung 24 eine Dialysierflüssigkeitspumpe 78 eingeschaltet, mit der die Dialysierflüssigkeit aus der einen Bilanzkammerhälfte in die andere Bilanzkammerhälfte der Bilanzkammer 32 gepumpt wird, wobei die flexible, nichtgezeigte Wand in der Bilanzkammer in entsprechender Weise verschoben wird.

Sofern ein Dialysegerät ohne Bilanzkammern verwendet wird, dient die Dialysierflüssigkeitspumpe 78 zur Förderung der Dialysierflüssigkeit von der nichtgezeigten Dialysierflüssigkeitsquelle durch den Dialysator 12 zum Abfluß.

Schließlich ist die Abzugsleitung 24 mit einem Druckmeßgerät 80 verbunden, so daß der im Dialysierflüssigkeitsweg 22 vorliegende Druck bestimmt werden kann.

Zur Steuerung der einzelnen Komponenten ist ein Steuergerät 82 vorgesehen, das über folgende Steuerleitungen mit den jeweiligen Komponenten verbunden ist:

1. Steuerleitung 84 zur Bilanzkammer 32 bzw.
2. und 3. Steuerleitungen 86 und 88 zu den 1. und 2. Absperrventilen 34 und 36,
4. Steuerleitung 90 zum Belüftungsventil 66,
5. Steuerleitung 92 zur Ultrafiltratpumpe 76,
6. Steuerleitung 94 zum Bypassventil 58,
7. Steuerleitung 96 zur Dialysierflüssigkeitspumpe 78.

Eine Signalleitung 98 geht von dem Druckmeßgerät 80 zum Steuergerät 82.

Sofern das Hämodialysegerät 10 nicht zur Behandlung eines Patienten benutzt wird, wird der Dialysator 12 entfernt, worauf die Spülung und Desinfektion des Geräts 10 erfolgt. Zu diesem Zweck werden die Zuleitung 22 und die Ableitung 24 vom Dialysator abgetrennt und miteinander mit Hilfe eines Verbindungsstücks 100 verbunden, wie dies in der Zeichnung strichliert dargestellt ist. Insofern stehen also dann die beiden Leitungen unmittelbar miteinander in Strömungsverbindung.

In den zweiten Zuleitungszweig 28 sind des weiteren ein nicht gezeigtes Dialysatorventil und stromauf hiervon eine Leitfähigkeitszelle als Sicherheitseinrichtung eingeschaltet, auf deren Signal hin beim Auftreten eines Fehlers das Bypassventil 58 geöffnet und das Dialysatorventil geschlossen wird.

Die in der Figur gezeigte Hämodialysemaschine wird während der Dialyse folgendermaßen betrieben:

Über die Bilanzkammer 32 wird zunächst frische Dialysierflüssigkeit dem Dialysator 12 zugeführt, wobei diese Zuführung mit Hilfe der Dialysierflüssigkeitspumpe 78 erfolgt. Die Dialysierflüssigkeit wird durch den ersten Zuleitungsabschnitt 26 in die erste Kammer 42 des Sterilfilters 38 gepumpt, wobei zunächst die Bypassleitung mit Hilfe des Bypassventils 58 verschlossen ist. Durch die Wirkung der Dialysierflüssigkeitspumpe 78 wird die Dialysierflüssigkeit durch die Membran 40 des Sterilfilters 38 gezogen, wobei die eventuell vorhandenen Pyrogene oder Keime an der Oberfläche der Membran 40 zurückgehalten werden. Die so sterilisierte Dialysierflüssigkeit gelangt durch den zweiten Zuleitungsabschnitt 22 in die erste Kammer 16 des Dialysators 12 und von dort in an sich bekannter Weise in die Ableitung 24, in der sowohl die Ultrafiltration als die Ausscheidung vonstatten geht.

Um zu verhindern, daß die Membran 40 sich durch Keime oder Pyrogene verstopft, wird in vorbestimmten Zeitabständen vom Steuergerät 82 ein Signal an das Bypassventil über die Steuerleitung 94 abgegeben, worauf das Bypassventil 58 geöffnet wird. Infolge des hohen Durchflußwiderstands des Dialysators 12 wird praktisch die gesamte Dialysierflüssigkeit nur noch durch die erste Kammer 42 des Sterilfilters 38 in die Bypassleitung 54 gefördert, wobei durch die Strömung der Dialysierflüssigkeit die an der Membranoberfläche befindlichen Keime oder Pyrogene abgeschert und mit dem Flüssigkeitsstrom fortgerissen werden. Es erfolgt so eine Reinigung der Membranoberfläche. Nach einem bestimmten Behandlungzeitraum wird dann durch das Steuergerät 82 das Bypassventil 58 erneut aktiviert, d. h. geschlossen, so daß die Dialysebehandlung wieder aufgenommen wird.

Nach der Dialyse wird der Dialysator 12 entfernt und die beiden Enden der Zuleitung 22 und der Abzugsleitung 24 mit einem Kurzschlußstück 100 verschlossen. Durch die Bilanzkammer 32 wird nun wechselweise eine übliche Desinfektionslösung bzw. Frischwasser zur Desinfizierung und Spülung des gesamten geschlossenen Systems zugeführt. Um beide Teile der Primär- und Sekundärseite des Sterilfilters 38 zu reinigen wird wiederum das Bypassventil 58 getaktet betrieben, so daß teilweise nur die erste Kammer 42 gespült und desinfiziert wird und teilweise auch die zweite Kammer 44 in den Spül- und Desinfektionsschritt einbezogen wird. Dabei ist in dieser Spül- und Desinfektionsphase die Ultrafiltrationspumpe 76 nicht in Betrieb, während die Dialysierflüssigkeitspumpe 78 betrieben ist. Ebenfalls ist das Belüftungsventil 66 bzw. die Belüftungseinrichtung 60 nicht in Betrieb.

Um das Sterilfilter 38 nach dem letzten Spülschritt auf seine Unversehrtheit zu überprüfen, führt das Steuergerät 82 folgende Steueroperationen durch:
Zunächst die Dialysierflüssigkeitspumpe 78 desaktiviert, so daß keine Zu- oder Abführung von Flüssigkeit erfolgt. Alternativ werden die beiden Absperrventile 34 und 36 geschlossen, d.h. es wird ein geschlossenes System aufgebaut. Des weiteren wird das Bypassventil 58 geschlossen und es wird das Belüftungsventil 66 geöffnet. Schließlich wird die Ultrafiltrationspumpe 76 in Betrieb genommen, wobei der sich am Druckmeßgerät 80 entstehende Druck beobachtet wird. Schließlich wird auch die Bilanzkammer 32 desaktiviert.

Nunmehr wird durch die Wirkung der Ultrafiltratpumpe 76 dem Stutzen 64, dem Inneren des Gehäuses 62, d.h. den beiden Kammer 70 und 72, der sich daran anschließenden Bypassleitung 54 und der ersten Kammer 42 des Sterilfilters 38 Luft zugeführt, wobei die Flüssigkeit durch die Poren der Membran 40 in die zweite Kammer 44 des Sterilfilters 38 verdrängt wird. Sobald samtliche Flüssigkeit aus der ersten Kammer 42 verdrängt worden ist, stellt die Membran 40, die mit Wasser benetzt ist, eine Barriere für ein weiteres Eindringen von Luft dar, wobei vorausgesetzt, daß die Membran intakt ist, also keine Risse oder Rupturen aufweist. So ist beispielsweise ein übliches Sterilfilter bei Überdrücken 1,5-3 bar für Luft (auf der Druckseite) undurchlässig. Ein entsprechender Unterdruck kann mit Hilfe des Druckmeßgeräts 80 bestimmt und als Signal dem Steuergerät 82 zugeführt werden, das nach Erreichen eines vorbestimmten Druckwerts die Ultrafiltratpumpe 76 desaktiviert und hierauf in zeitlicher Folge die vom Druckmeßgerät 80 abgegebenen Druckwerte registriert. Über einen bestimmten Zeitraum, beispielsweise einige Minuten bleibt der sich am Druckmeßgerät 80 einstellende Unterdruck in etwa konstant. Sobald eine vorbestimmte Zeitspanne ohne wesentliche Veränderung dieser Druckwerte abgelaufen ist, meldet das Steuergerät 82 das Sterilfilter 38 als intakt bzw. fordert den Anwender auf, das Sterilfilter 38 auszuwechseln und den neuen Filter einer weiteren Spül- und Desinfektionsphase zu unterziehen.

Nach der Prüfung ist die gesamte Anordnung für einen weiteren Einsatz betriebsbereit.

**Patentansprüche**

1. Hämodialysevorrichtung mit Sterilisiereinrichtung für die Dialysierflüssigkeit, mit einem Dialysator (12), der durch eine Membran (14) in zwei Kammern (16, 18) geteilt ist, wobei die erste Kammer (16) in einen Dialysierflüssigkeitsweg und die zweite Kammer (18) in einen Blutweg geschaltet ist und der Dialysierflüssigkeitsweg eine Zuleitung (22), die sich von einer Einrichtung zur Bereitstellung von Dialysierflüssigkeit bis zum Dialysator erstreckt und in die ein erstes Absperrorgan (34) eingeschaltet ist, und eine Ableitung (24) aufweist, die sich vom Dialysator zum Abfluß erstreckt und in die ein zweites Absperrorgan (36) eingeschaltet ist, mit einer Pumpe (78) zum Fördern der Dialysierflüssigkeit im Dialysierflüssigkeitsweg, einer zwischen den Absperrorganen im Dialysierflüssigkeitsweg vorgesehenen Ultrafiltrationseinrichtung (74, 76), mit einer die Zuleitung mit der Ableitung des Dialysierflüssigkeitswegs verbindenden Bypassleitung (54), in die ein Bypassventil (58) eingschaltet ist, und mit einem Sterilfilter (38), das durch eine Keime zurückhaltende Membran (40) in eine erste Kammer (42) und eine zweite Kammer (44) geteilt ist, mit einem ersten Abschnitt (26) der Zuleitung der Dialysierflüssigkeit zur ersten Kammer des Sterilfilters und einem zweiten Abschnitt (28) der Zuleitung der Dialysierflüssigkeit von der zweiten Kammer des Sterilfilters zum Dialysator, dadurch gekennzeichnet, daß der Auslaß (48) der ersten Kammer (42) des Sterilfilters (38) mit der zur Ableitung (24) führenden Bypassleitung (54) verbunden ist.

2. Vorrichtung nach Anspruch 1, dadurch gekennzeichnet, daß in die Bypassleitung (54) zwischen dem Bypassventil (58) und der ersten Kammer (42) des Sterilfilters (38) eine Belüftungseinrichtung (60) eingeschaltet ist.

3. Vorrichtung nach Anspruch 2, dadurch gekennzeichnet, daß die Belüftungseinrichtung (60) ein Belüftungsventil (66) aufweist.

4. Vorrichtung nach Anspruch 3, dadurch gekennzeichnet, daß die Belüftungseinrichtung (60) ein Gehäuse (62) aufweist, das in die Bypassleitung (54) eingeschaltet ist und in seinem Innenraum eine mikroporöse, hydrophobe Membran (68) aufweist, die den Innenraum in eine erste Kammer (70), die mit einem vom Gehäuse abgehenden Stutzen (64) in Strömungsverbindung steht, und eine zweite Kammer (72) teilt, die mit der Bypassleitung (54) in Strömungsverbindung steht.

5. Vorrichtung nach Anspruch 4, dadurch gekennzeichnet, daß das Ende des Stutzens (64) mit dem Belüftungsventil (66) verbunden ist.

6. Vorrichtung nach Anspruch 5, gekennzeichnet dadurch, daß die Absperrorgane als Bilanzkammer (32) oder als Absperrventile (36 und 34) ausgebildet sind, sowie durch ein Steuergerät (82), das über eine erste Steuerleitung (84) mit der Bilanzkammer (32) oder zweite und dritte Steuerleitungen (86 und 88) mit den ersten und zweiten Absperrventilen (36 und 34), über eine vierte Steuerleitung (90) mit dem Belüftungsventil (66), über eine fünfte Steuerleitung (92) mit einer Ultrafiltratpumpe (76), über eine sechste Steuerleitung (84) mit dem Bypassventil (58), über eine siebte Steuerleitung (96) mit der Dialysierflüssigkeitspumpe (78) und über eine Signalleitung (98) mit einem Druckmeßgerät (80) verbunden ist.

7. Verfahren zur Reinigung des Sterilfilters (38) einer Hämodialysevorrichtung nach Anspruch 6, dadurch gekennzeichnet, daß das Steuergerät (82) das Bypassventil (58) in vorbestimmten Zeitabständen öffnet und schließt.

8. Verfahren zur Überprüfung des Sterilfilters (38) einer Hämodialysevorrichtung nach Anspruch 6, dadurch gekennzeichnet, daß der Dialysator (12) entfernt wird, die Zuleitung (22) und die Ableitung (24) mit einem Verbindungsstück (100) verschlossen werden und daß das Steuergerät (82) die Dialysierflüssigkeitspumpe (78) desaktiviert, die Bilanzkammer (32) oder die Absperrventile (34 und 36) verschließt, die Belüftungseinrichtung (60) gegenüber der Atmosphäre öffnet, die Ultrafiltratpumpe (76) solange in Betrieb setzt, bis ein vorbestimmter Druckwert vom Druckmeßgerät (80) signalisiert wird, anschließend die Ultrafiltratpumpe (76) außer Betrieb setzt, die vom Druckmeßgerät (80) abgegebenen Druckwertsignale über einen vorbestimmten Zeitraum überwacht und anschließend die Intaktheit des Sterilfilters (38) anzeigt.

**Claims**

1. Hemodialysis apparatus comprising sterilizing means for the dialysis solution, a dialyser (12) divided by a membrane (14) into two chambers (16, 18), the first chamber (16) being connected into a dialysis solution path and the second chamber (18) into a blood path, the dialysis solution path comprising a supply line (22) which extends from a means for preparing dialysis solution to the dialyzer and into which a first shutoff member (34) is connected and an exit line (24) which extends from the dialyzer to the discharge and into which a second shutoff member (36) is connected, and comprising furthermore a pump for conveying the dialysis solution in the dialysis solution path, an ultrafiltration means (74, 76) provided between the shutoff members in the dialysis solution path, a bypass line (54) which connects the supply line to the exit line of the dialysis solution path and into which a bypass valve (58) is connected, and a sterile filter (38) which is divided by a germ-retaining membrane (40) into a first chamber (42) and a second chamber (44), with a first portion (26) of the supply line of the dialysis solution to the first chamber of the sterile filter and a second portion (28) of the supply line of the dialysis solution from the second chamber of the sterile filter to the dialyzer, characterized in that the outlet (48) of the first chamber (42) of the sterile filter (38) is connected to the bypass line (54) leading to the exit line (24).

2. Apparatus according to claim 1, characterized in that a ventilation means (60) is connected into the bypass line (54) between the bypass valve (58) and the first chamber (42) of the sterile filter (38).

3. Apparatus according to claim 2, characterized in that the ventilation means (60) comprises a ventilation valve (66).

4. Apparatus according to claim 3, characterized in that the ventilation means (60) comprises a housing (62) which is connected into the bypass line (54) and comprises in its interior a microporous hydrophobic membrane (68) which divides the interior into a first chamber (70) in flow communication with a tube connecting piece (64) leading from the housing and a second chamber (72) in flow communication with the bypass line (54).

5. Apparatus according to claim 4, characterized in that the end of the tube connecting piece (64) is connected to the ventilation valve (66).

6. Apparatus according to claim 5, characterized in that the shutoff means are formed as balance chamber (32) or shutoff valves (36 and 34) and by a control unit (82) which is connected via a first control line (84) to the balance chamber (32) or second and third control lines (86 and 88) to the first and second shutoff valves (36 and 34), via a fourth control line (90) to the ventilation valve (66), via a fifth control line (92) to an ultrafiltrate pump (76), via a sixth control line (84) to the bypass valve (58), via a seventh control line (96) to the dialysis solution pump (78) and via a signal line (98) to the manometer (80).

7. Method of cleaning the sterile filter (38) of a hemodialysis apparatus according to claim 6, characterized in that the control unit (82) opens and closes the bypass valve (58) at predetermined intervals of time.

8. Method of checking the sterile filter (38) of a hemodialysis apparatus according to claim 6, characterized in that the dialyzer (12) is removed, the supply line (22) and the exit line (24) are closed with a connector (100) and that the control unit (82) deactivates the dialysis solution pump (78), closes the balance chamber (32) or the shutoff valves (34 and 36), opens the ventilation (60) with respect to the atmosphere, sets the ultrafiltrate pump (76) in operation until a predetermined pressure value is reported by the manometer (80), thereafter sets the ultrafiltrate pump (76) out of operation, monitors the pressure value signals furnished by the manometer (80) for a predetermined period of time and subsequently indicates the intactness of the sterile filter (38).

**Revendications**

1. Dispositif d'hémodialyse avec équipement de stérilisation pour le liquide de dialyse, comportant un dialyseur (12) qui est partagé en deux chambres (16, 18) par une membrane (14), dans lequel la première chambre (16) est montée dans un parcours de liquide de dialyse et la seconde chambre (18) dans un parcours sanguin et le parcours de liquide de dialyse comporte une conduite d'arrivée (22) qui s'étend depuis un dispositif de préparation du liquide de dialyse jusqu'au dialyseur et dans laquelle est monté un premier organe d'arrêt (34), ainsi qu'une conduite de départ (24) qui s'étend du dialyseur jusqu'à l'évacuation et dans laquelle est monté un second organe d'arrêt (36), comportant une pompe (78) pour la circulation du liquide de dialyse dans le parcours de liquide de dialyse, un dispositif d'ultrafiltration (74, 76), prévu entre les organes d'arrêt dans le parcours de liquide de dialyse, comportant une conduite de dérivation (54) reliant la conduite d'arrivée à la conduite de départ du parcours de liquide de dialyse, dans laquelle est montée une soupape de dérivation (58) et comportant un filtre stérile (38) qui est partagé, par une membrane (40) retenant les germes, en une première chambre (42) et une seconde chambre (44), comportant une première partie (26) de la conduite d'arrivée du liquide de dialyse vers la première chambre du filtre stérile et une seconde partie (28) de la conduite d'arrivée du liquide de dialyse de la seconde chambre du filtre stérile au dialyseur, caractérisé en ce que la sortie (48) de la première chambre (42) du filtre stérile (38) est reliée à la conduite de dérivation (54) menant à la conduite de départ (24).

2. Dispositif selon la revendication 1, caractérisé en ce qu'un dispositif d'aération (60) est monté dans la conduite de dérivation (54), entre la soupape de dérivation (58) et la première chambre (42) du filtre stérile (38).

3. Dispositif selon la revendication 2, caractérisé en ce que le dispositif d'aération (60) comporte une soupape d'aération (66).

4. Dispositif selon la revendication 3, caractérisé en ce que le dispositif d'aération (60) comporte un boîtier (62) qui est monté dans la conduite de dérivation (54) et comporte à l'intérieur une membrane (68) hydrophobe, microporeuse qui partage l'intérieur en une première chambre (70) qui est en liaison d'écoulement avec une tubulure (64) partant du boîtier, et en une seconde chambre (72) qui est en liaison d'écoulement avec la conduite de dérivation (54).

5. Dispositif selon la revendication 4, caractérisé en ce que l'extrémité de la tubulure (64) est reliée à la soupape d'aération (66).

6. Dispositif selon la revendication 5, caractérisé en ce que les organes d'arrêt sont conçus sous la forme de chambres d'équilibrage (32) ou de robinets d'arrêt (36 et 34), ainsi que par un appareil de commande (82) qui est relié avec la chambre d'équilibrage (32) par une première ligne de commande (84) ou avec les premier et second robinets d'arrêt (36 et 34) par une deuxième et une troisième lignes de commande (86 et 88), avec la soupape d'aération (66) par une quatrième ligne de commande (90), avec une pompe d'ultrafiltration (76) par une cinquième ligne de commande (92), avec la soupape de dérivation (58) par une sixième ligne de commande (84), avec la pompe de liquide de dialyse (78) par une septième ligne de commande (96) et avec un appareil de mesure de pression (80), par une ligne de signalisation (98).

7. Procédé pour le nettoyage du filtre stérile (38) d'un dispositif d'hémodialyse selon la revendication 6, caractérisé en ce que l'appareil de commande (82) ouvre et ferme la soupape de dérivation (58) à intervalles de temps prédéterminés.

8. Procédé pour la vérification du filtre stérile (38) d'un dispositif d'hémodialyse selon la revendication 6, caractérisé en ce que le dialyseur (12) est enlevé, la conduite d'arrivée (22) et la conduite de départ (24) sont fermées par une pièce de liaison (100) et en ce que l'appareil de commande (82) désactive la pompe de liquide de dialyse (78), ferme la chambre d'équilibrage (32) ou les robinets d'arrêt (34 et 36), ouvre le dispositif d'aération (60) par rapport à l'atmosphère, met en service la pompe d'ultrafiltration (67) jusqu'à ce qu'une pression prédéterminée soit signalée par l'appareil de mesure de pression (80), puis met la pompe d'ultrafiltration (76) hors service, contrôle les signaux de valeur de pression délivrés par l'appareil de mesure de pression (80) pendant un intervalle de temps prédéterminé puis indique que le filtre stérile (38) est intact.